# EUROPÄISCHE PATENTSCHRIFT

(11) **EP 4 236 780 B1**
(45) Veröffentlichungstag und Bekanntmachung des Hinweises auf die Patenterteilung: **28.08.2024**
(21) Anmeldenummer: 21802328.1
(22) Anmeldetag: 01.11.2021
(51) Int. Cl.: A61B 5/00, A61B 7/04, A61B 5/024

(54) **GERÄT ZUR DETEKTION VON HERZTON-SIGNALEN EINES BENUTZERS UND VERFAHREN DAZU**
DEVICE FOR DETECTING HEART SOUND SIGNALS OF A USER AND METHOD THEREFOR
DISPOSITIF DE DÉTECTION DE SIGNAUX SONORES CARDIAQUES D'UN UTILISATEUR ET PROCÉDÉ ASSOCIÉ

(30) Priorität: 30.10.2020 CH 13962020
(43) Veröffentlichungstag der Anmeldung: 06.09.2023
(73) Patentinhaber: Rocket Science AG, 8045 Zürich (CH)
(72) Erfinder: FRICK, Christian, 8038 Zürich (CH); NIQUILLE, Philippe, 6300 Zug (CH)
(74) Vertreter: Zwick, Evelyn
(86) Internationale Anmeldenummer: PCT/EP2021/080258
(87) Internationale Veröffentlichungsnummer: WO 2022/090535

(56) Entgegenhaltungen:
- CN-A- 104 244 126
- KR-A- 20200 075 737
- US-A- 5 721 783
- US-A1- 2008 146 890
- US-A1- 2010 189 269
- US-A1- 2019 247 010
- US-B2- 8 705 784

## Beschreibung

### Technisches Gebiet

Die Erfindung betrifft ein Gerät und ein Verfahren zur Erzeugung von aufbereiteten Herzton-Signalen eines Benutzers auf Grund eines akustischen Ausgangssignals, um eine Herztätigkeit des Benutzers analysieren zu können, wobei das Gerät im Bereich eines Ohres des Benutzers tragbar ist, umfassend einen Verstärker und einen ausgangsseitig damit verbundenen Lautsprecher, zur Ausgabe eines Referenzsignals in einen Gehörgang des Benutzers, ein Mikrophon zum Aufnehmen eines Ausgangssignals in diesem Gehörgang des Benutzers, welches das Referenzsignal und die damit modulierten Herztöne des Benutzers umfasst, sowie einen Prozessor zur Echtzeit-Bearbeitung dieses Ausgangssignals, zur Erzeugung der aufbereiteten Herzton-Signale.

### Technischer Hintergrund

Kopfhörer, insbesondere Ohrhörer, sogenannte In-ear-Systeme, haben in den letzten Jahren stark an Bedeutung und Beliebtheit gewonnen, während ihre Qualität auch stets verbessert wurde. So wurden beispielsweise auch Kopfhörer mit «Active Noise Cancelling» (ANC) entwickelt, welche Aussengeräusche eliminieren. Mit diesen können Benutzer Stille geniessen, selbst wenn sie Lärm ausgesetzt sind. Heutzutage tragen zahlreiche Leute während mehreren Stunden täglich Kopfhörer, wie Mitarbeiter in Callcentern, Passagiere im öffentlichen Verkehr, Sportler oder Lernende, um nur einige zu nennen. Die dabei empfangenen Audiosignale, seien es Gespräche, Musik, Radio, Hörspiele oder Hörbücher, dienen dem Wohlbefinden, dem Genuss, zur Entspannung, der Abschottung gegen Aussen oder der Informationsbeschaffung.

Aus der US 8705784 ist ein Kopfhörer in Form eines Ohrhörers bekannt, der mit Hilfe eines Detektors bestimmen kann, ob der Ohrhörer am Ohr des Benutzers angelegt ist oder nicht. Sobald der Detektor erkennt, dass der Ohrhörer abgelegt wurde, wird automatisch die Tonausgabe über Lautsprecher gestoppt, um Energie zu sparen. Der Detektor umfasst dazu ein Mikrophon und kann prüfen, ob eine Signalkomponente des erfassten Tonsignals Herzschlaggeräuschen, der Atmung oder einem Sprechen des Benutzers zugeordnet werden kann. Dazu wird von dem Signal, das vom Lautsprecher abgegeben wurde, das vom Mikrofon erfasste Signal subtrahiert und die Differenz davon in einen Detektor geleitet. Dieser soll erkennen, ob Komponenten von Herzschlägen, von einer Atmung und/oder vom Sprechen eines Benutzers erkannt werden. Auf diese Weise können Herzschläge derart rudimentär erfasst werden, dass eine binäre Entscheidung gefällt werden kann, ob der Kopfhörer am Ohr ist oder nicht.

Es gibt auch andere, ähnliche Systeme, welche auf diese Weise Herzschlaggeräusche feststellen können. Genauere Daten lassen sich mit solchen Kopfhörern allerdings nicht ermitteln.

In der KR20200075737A wird ein System beschrieben, wobei anstatt akustischer Signale Impedan Signale verwendet werden, um Störsignale aus der Umwelt auszuschliessen. Somit werden keine Tön mittels Lautsprecher in den Körper abgegeben und auch keine Töne am Ohr mittels Mikrophon aufgenommen.

In der CN104244126A wird vorgeschlagen, eine Kavität im Kopfhörer vorzusehen und ein erste Mikrophon dort, störungsfrei von Aussengeräuschen, einzubauen, welches Herztonsignale erfassen kann. Ein zweites Mikrofon wäre im Kopf des Ohrhörers installiert. Das damit erfasste Signal soll durch Filterung eine Schätzung des ursprünglichen Signals liefern, ohne Herzfunktion. Die Differenz dieser beiden von den Mikrophonen erfassten Signalen soll schliesslich auf die Herzfrequenz schliessen lassen. Auch hier wird lediglich die Frequenz als Ergebnis ausgegeben. Keine genaueren Daten werden ausgegeben.

### Darstellung der Erfindung

Es ist die Aufgabe der vorliegenden Erfindung, ein am Ohr tragbares Gerät zu beschreiben, welches nicht nur Herzschlaggeräusche erfassen und identifizieren kann, sondern auch aus den erfassten Geräuschen aufbereitete Herzton-Signale des Benutzers erstellen kann, auf Grund dessen Aussagen über den Gesundheits- und/oder Fitnesszustand möglich sind. Dies soll mit herkömmlichen Kopfhörern möglich sein, die in erster Linie für die Übertragung von Audiosignalen genutzt werden, oder mit Hörgeräten, welche Benutzer mit Hörschwächen tragen. Solche Kopfhörer sollen vorzugsweise auch kabellos betreibbar sein, um die Bewegungsfreiheit der Benutzer nicht einzuschränken.

Eine weitere Aufgabe besteht darin, ein Verfahren zu beschreiben, wie mit einem solchen Gerät Daten erfasst und derart aufbereitet werden können, dass dadurch aufbereitete Herzton-Signale zur Überprüfung der Herztätigkeit erstellt werden können.

Die Aufgaben werden mit den Merkmalen der unabhängigen Ansprüche der jeweiligen Anspruchskategorie gelöst.

Erfindungsgemäss umfasst das Gerät dazu zusätzlich einen Tongenerator zur Erzeugung eines Referenzsignals, welches ein Sinussignal im Ultraschallbereich ist und sowohl in den Verstärker als auch in den Prozessor geleitet werden kann. Im Prozessor ist zudem ein Vorfilter angeordnet, welcher aus dem Ausgangssignal entstörte Herzton-Signale erzeugen kann, sowie mindestens ein Multiplikator, der die entstörten Herzton-Signale mit dem Referenzsignal demodulieren und so ein Zwischensignal erzeugen kann. Zudem umfasst der Prozessor ein Programm zur Bildung von Mittelwerten, welches aus dem Zwischensignal verbesserte Herzton-Signale bilden kann und einen frequenzselektiven Filter, welcher aus diesen verbesserten Herzton-Signalen die aufbereiteten Herzton-Signale erzeugen kann.

Die Qualität der mit diesem Gerät erzeugbaren aufbereiteten Herzton-Signale übertrifft jene von Signalen, die mit herkömmlichen Geräten erzeugt werden können, derart stark, dass qualitative Aussagen über die Herztöne gemacht werden können. Als Qualität wird in dieser Schrift jeweils Genauigkeit, Zuverlässigkeit, Empfindlichkeit und/oder Störungsanfälligkeit der Signale verstanden.

Die so erhaltenen aufbereiteten Herzton-Signale enthalten nur noch relevante Informationen, sodass sie als Daten über eine Ausgabeeinheit an eine externe Datenverarbeitungsanlage, beispielsweise ein Smartphone, als Auswertegerät verschickt werden können. Zusätzlich oder alternativ dazu können die erhaltenen aufbereiteten Herzton-Signale im Prozessor selbst analysiert werden, in einem dazu vorgesehenen Herzton-Analyseprogramm. Durch Vergleiche mit Sollwerten kann beispielsweise auf die Herztätigkeit und somit auf gewisse Gesundheitsrisiken und/oder auf den Fitnesszustand des Benutzers geschlossen werden. Auf Grund der Resultate dieser Analyse kann der Benutzer durch einen Lautsprecher am Gerät entsprechend informiert werden. Bei Herzrasen oder bei einem bevorstehenden Herzinfarkt kann, beispielsweise durch den Lautsprecher, die Nachricht vermittelt werden: «Bitte setzen Sie sich hin und stoppen Sie unverzüglich alle Aktivitäten, durch die Sie oder andere gefährdet werden können. Holen Sie Hilfe oder alarmieren Sie den Notruf.» Ähnliche Nachrichten sind beispielsweise für sportliche Betätigung denkbar, indem der Benutzer in einer Übung aufgrund seiner Herztätigkeit individuell angeleitet wird. Oder der Benutzer wird während dem Büroalltag zu gezielten Bewegungsübungen animiert, ähnlich wie dies von Armbanduhren mit Fitness Funktion bereits bekannt ist.

Das erfindungsgemässe Verfahren zur Erzeugung von aufbereiteten Herzton-Signalen eines Benutzers auf Grund eines akustischen Ausgangssignals, zur Überwachung der Herztätigkeit des Benutzers, wird unter Verwendung eines oben beschriebenen Gerätes durchgeführt und umfasst folgende Schritte in der genannten Reihenfolge:
- Positionieren des Gerätes im Bereich eines Ohrs des Benutzers;
- Erzeugung, Verstärkung und Ausgabe eines Referenzsignals als Sinussignal im Ultraschallbereich, in einen Gehörgang des Benutzers mittels eines Tongenerators, eines Verstärkers und eines Lautsprechers;
- Aufnehmen eines akustischen Ausgangssignals im Gehörgang des Benutzers welches dessen Herztöne und das Referenzsignal umfasst, mit einem Mikrophon;
- Weiterleiten dieses Ausgangssignals an einen Prozessor;
- Erzeugung von aufbereiteten Herzton-Signalen durch Echtzeit Bearbeitung dieses Ausgangssignals im Prozessor, indem
   ∘ in einem Vorfilter aus dem Ausgangssignal entstörte Herzton-Signale erzeugt werden,
   ∘ in einem Multiplikator die entstörten Herzton-Signale mit dem Referenzsignal demoduliert werden und so ein Zwischensignal erzeugt wird,
   ∘ in einem Programm zur Bildung von Mittelwerten aus dem Zwischensignal verbesserte Herzton-Signale gebildet werden,
   ∘ und in einem frequenzselektiven Filter aus den verbesserten Herzton-Signalen die aufbereiteten Herzton-Signale erzeugt werden.

Die aufbereiteten Signale können anschliessend weiterverarbeitet und/oder mit mindestens einem Mittel weitergeleitet werden.

In den abhängigen Ansprüchen sind weitere bevorzugte Vorrichtungen und Verfahren beschrieben. Mit diesen können Herzton-Signale mit einer bisher unerreichten Qualität erzeugt werden, bei Verwendung von herkömmlichen Mitteln. So können Leute, die beispielsweise ein Hörgerät tragen, ständig auf allfällige Herzstörungen überwacht werden, ohne dass sie dazu ein zusätzliches Gerät brauchen. Bei einem Herzproblem kann einerseits der Benutzer selbst gewarnt werden, damit er sich zum Beispiel hinsetzt, um sich nicht bei einem Sturz zu verletzen, oder, falls er am Steuer eines Fahrzeuges sitzt, noch seitlich anhalten und den Warnblinker einschalten, um Unfälle zu vermeiden. Andererseits kann ein externes Gerät wie ein Smartphone im Fall eines Herzproblems selbstständig den Notruf verständigen und die genaue Position des Benutzers durchgeben. Eine schnelle medizinische Hilfe kann bei Herzproblemen lebensrettend sein.

Eine breitere Anwendung kann allerdings bei sportlichen Betätigungen erreicht werden, indem Fitnessübungen in ihrer Dauer und Anstrengung an die körperliche Verfassung des Benutzers angepasst werden.

### Kurze Erläuterung zu den Figuren

Die Erfindung soll nachfolgend im Zusammenhang mit den Zeichnungen näher erläutert werden. Es zeigen:
- Fig. 1: eine schematische Darstellung des erfindungsgemässen Gerätes im Betrieb;
- Fig. 2: eine schematische Darstellung des Gerätes in Verbindung mit einem Zweitgerät und/oder mit einer Datenverarbeitungsanlage;
- Fig. 3: eine schematische Darstellung des Prozessors;
- Fig. 4: a) akustisches Ausgangssignal; b) entstörte Herzton-Signale; c) aufbereitete Herzton-Signale.

### Wege zur Ausführung der Erfindung

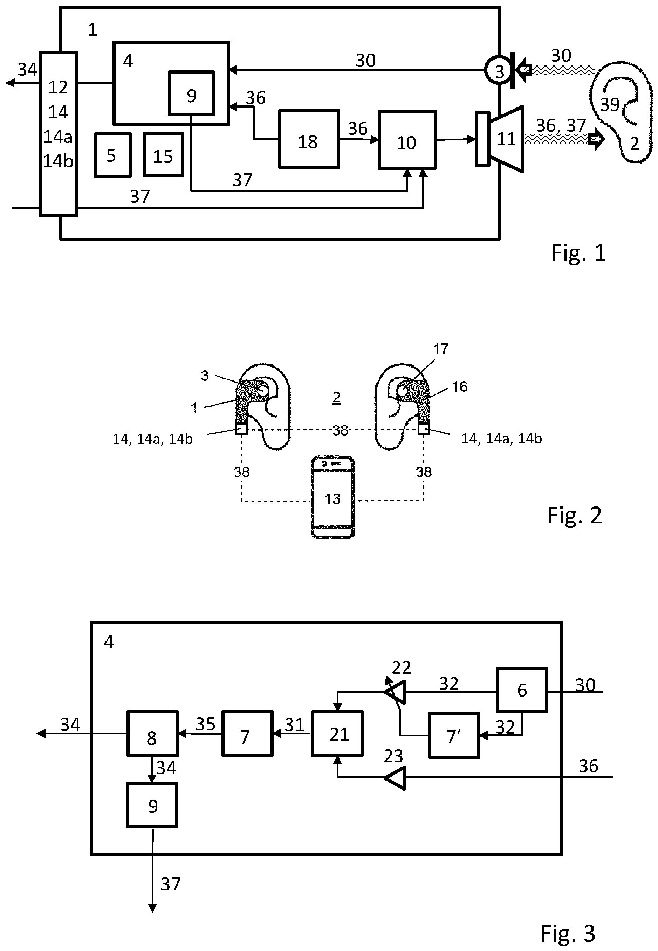

In Fig. 1 ist ein erfindungsgemässes Gerät 1 dargestellt zur Erzeugung von aufbereiteten Herzton-Signalen 34 eines Benutzers 2, wobei vom Benutzer 2 lediglich eines seiner Ohren schematisch dargestellt ist. Das Gerät 1 in Fig. 1 ist symbolisch in Rechteckform dargestellt, wobei es in Realität als Kopfhörer, Ohrhörer, auch In-ear-System genannt, als Hörgerät oder dergleichen ausgestaltet ist, sodass es am Kopf, im Bereich des Ohres oder im Ohr selbst tragbar ist, wie in Fig. 2 ersichtlich. Es kann zudem durch eine Ausgabeeinheit 12 mit einer externen Datenverarbeitungsanlage 13 verbunden sein oder drahtlos mittels Sender und Empfänger 14 damit kommunizieren. Die aufbereiteten Herzton-Signale 34 werden auf Grund eines akustischen Ausgangssignals 30 erzeugt, um eine Herztätigkeit des Benutzers 2 analysieren zu können.

Das Gerät 1 umfasst gemäss Fig. 1 einen Tongenerator 18 zur Erzeugung eines Referenzsignals 36, welches ein Sinussignal im Ultraschallbereich ist. Dieses wird einen Verstärker 10 und einen ausgangsseitig damit wiederum verbundenen Lautsprecher 11 geleitet, zur Ausgabe des Referenzsignals 36 in einen Gehörgang des Benutzers 2. Zudem umfasst das Gerät 1 ein Mikrophon 3 zum Aufnehmen eines Ausgangssignals 30 in diesem Gehörgang des Benutzers 2, welches das Referenzsignal 36 und die damit modulierten Herztöne 39 des Benutzers 2 umfasst. Zudem umfasst das Gerät 1 einen Prozessor 4 zur Erzeugung der aufbereiteten Herzton-Signale 34 durch Echtzeit-Bearbeitung aus dem Ausgangssignal 30 und dem Referenzsignal 36.

Der Prozessor 4 kann auch ein Herzton-Analyseprogramm 9 umfassen zur Analyse der aufbereiteten Herzton-Signale 34 auf Grund von gespeicherten Vergleichswerten. Daraus kann es ein zusätzliches Signal 37 erzeugen, welches wiederum in den Verstärker 10 und anschliessend in den Lautsprecher 11 geleitet werden kann, um den Benutzer 2 auf geeignete Weise über die Analyse zu informieren.

Alternativ kann die Analyse in einem Herzton-Analyseprogramm 9 auch auf einer externen Datenverarbeitungsanlage 13 durchgeführt werden und als zusätzliches Signal 37 in das Gerät 1 eingespielt werden. Es kann anschliessend über den Verstärker 10 und den Lautsprecher 11 abgespielt werden, um dem Benutzer 2 Anweisungen oder Informationen zukommen zu lassen.

Weitere zusätzliche Signale 37 können in das Gerät 1 eingehen und ebenfalls über Verstärker 10 und Lautsprecher 11 in den Gehörgang den Benutzers 2 abgespielt werden, beispielsweise Musik oder Telefongespräche.

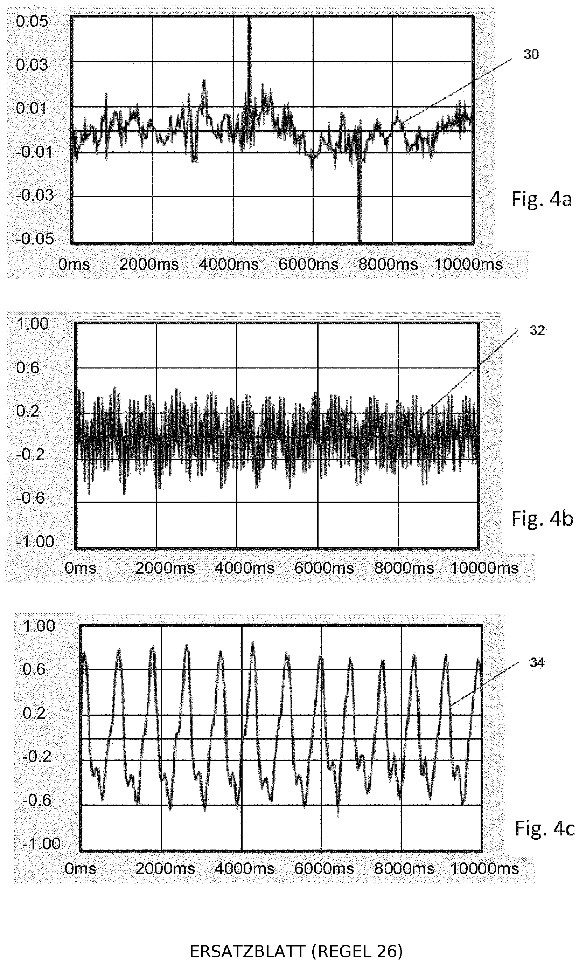

Die Signalverarbeitung geschieht im Prozessor 4, der in Fig. 3 dargestellt ist. Das vom Mikrophon 3 erfasste akustische Ausgangssignal 30, dargestellt in Fig. 4a, wird zu einem Vorfilter 6 geleitet, der sich im Prozessor 4 befindet. Dieser Vorfilter 6 eliminiert im Gebrauch Störsignale und schafft dadurch entstörte Herzton-Signale 32, wie sie in Fig. 4b dargestellt sind.

Diese entstörten Herzton-Signale 32 gelangen anschliessend im Prozessor 4, zusammen mit dem Referenzsignal 36 aus dem Tongenerator 18, in einen Multiplikator 21. Sowohl die entstörten Herzton-Signale 32 als Referenzsignal 36 können zudem in einem ersten resp. zweiten Signalverstärker 22, 23 verstärkt werden. Der Multiplikator 21 vollzieht auf Grund seiner eingegangenen Signale eine Demodulation des Ultraschallsignals und leitet schliesslich neue Herzton-Signale 31 in ein Programm 7 zur Bildung von Mittelwerten. Von den verschiedenen Methoden zur Mittelung von Signalen und anderen Daten wird hier bevorzugt die RMS Mittelung, gleitende Mittelung des Betrags oder gleitende Mittelung angewandt. Aus diesem Programm 7 werden schliesslich verbesserte Herzton-Signale 35 an einen frequenzselektiven Filter 8 zur Aufbereitung ausgegeben, der schliesslich die gewünschten aufbereiteten Herzton-Signale 34 liefert, dargestellt in Fig. 4c. Diese aufbereiteten Herzton-Signale 34 werden anschliessend in direkter oder indirekter Form von mindestens einem der Mittel 11, 12 weitergeleitet. Sie können beispielsweise durch die Ausgabeeinheit 12 direkt an eine Datenverarbeitungsanlage 13 übermittelt werden, die beispielsweise als Smartphone oder PC ausgestaltet sein kann.

Zur Verbesserung der Qualität kann der aufbereiteten Herzton-Signale 34 ein zweites Programm 7' zur Bildung von Mittelwerten angeordnet werden, welches Mittelwerte aus den entstörten Herzton-Signalen 32 bildet, zur Pegeleinstellung des ersten Signalverstärkers 22.

Aus den aufbereiteten Herzton-Signalen 34 lassen sich nicht nur die Herztöne des Benutzers 2 genau ermitteln, es lassen sich mit dem optionalen Herzton-Analyseprogramm 9, das im Gerät 1 selbst und/oder in der Datenverarbeitungsanlage 13 angeordnet sein kann, auch weitere Informationen über den Gesundheitszustand des Benutzers ermitteln. Diese können als zusätzliches Signal 37 schliesslich durch den Lautsprecher 11 ausgegeben werden.

Im Herzton-Analyseprogramm 9 kann eine weitere Verarbeitung und Analyse der Daten erfolgen, insbesondere eine Spektralanalyse oder eine Spektralanalyse der Autokorrelation. Es können auch Vergleiche mit Sollwerten vorgenommen werden, wobei diese Sollwerte Standardwerte oder für den Benutzer individualisierte Werte sein können. Die erfassten Daten können auch für medizinische oder sporttechnische Studien gesammelt und ausgewertet werden.

Zudem können weitere Aktionen folgen, wie Ratschläge, wie sich der Benutzer verhalten soll. So können Turn- oder Yogaübungen angepasst werden, gemäss dem festgestellten Erschöpfungsgrad des Benutzers 2, ohne dass dieser im Detail darüber informiert werden muss. In Extremfällen kann auch Hilfe angefordert werden, wenn sich beispielsweise abzeichnet, dass der Benutzer 2 in Kürze einen Zusammenbruch erleiden könnte.

Die Ausgabeeinheit 12 des Gerätes 1 umfasst vorzugsweise einen Sender 14a zur kabellosen Übertragung auf die externe Datenverarbeitungsanlage 13. Auch ein Empfänger 14b kann vorteilhaft sein, zum Erhalten von Informationen von der externen Datenverarbeitungsanlage 13. Diese Information kann im Gerät 1 auch einem anderen Zweck dienen, beispielsweise dem Abspielen von Musik.

Entscheidend ist, dass im Prozessor 4 aus dem umfangreichen Datenpaket des akustischen Ausgangssignals 30 gemäss Fig. 4a eine massive Datenreduktion vorgenommen wird, um mit den aufbereiteten Herzton-Signalen 34 gemäss Fig. 4c nur noch einen Bruchteil des ursprünglichen Datenvolumens übertragen zu müssen.

Im Gegensatz zu einer Vorrichtung nach dem Stand der Technik, wie diese in der US 8705784 beschrieben ist, ist die Qualität der aufbereiteten Herzton-Signale 34 derart hoch, dass nicht nur eine binäre Feststellung ausgelesen werden kann, ob die Kopfhörer am Ohr des Benutzers 2 getragen werden oder nicht, sondern eine Analyse zu differenzierten Aussagen führen kann. Dies liegt massgebend an der angewandten Ultraschallmodulations- und -demodulationsmethode, sowie an der numerischen Auswertung mittels Mittelwerten 7, 7' und frequenzselektiven Filtern 8.

Vorzugsweise umfasst das Gerät 1 einen Datenspeicher 5 und/oder einen Energiespeicher 15, beispielsweise als austauschbare und/oder aufladbare Batterie. Auf dem Datenspeicher 5 können eine Auswahl an möglichen Nachrichten zum Wiedergeben gespeichert sein und/oder Referenzwerte für eine Analyse. Der Energiespeicher 15 kann den Prozessor 4 und den Verstärker 10 und/oder weitere Komponenten speisen.

In einer bevorzugten Anordnung kann dem Gerät 1 ein Zweitgerät 16 derselben Art, das am zweiten Ohr des Benutzers 2 tragbar ist und ein zweites Mikrophon 17 umfasst, zugeordnet sein. In Fig. 2 ist eine solche Anordnung dargestellt. Daten 38 können mittels Kabeln oder kabellos mittels Sendern 14a und Empfängern 14b entweder direkt vom Zweitgerät 16 an das Gerät 1 übermittelt werden, oder sie können vom Gerät 1 und vom Zweitgerät 16 an eine externe Datenverarbeitungsanlage 13 gesendet werden. Im einfachsten Fall reicht ein Sender 14a beim Zweitgerät 16, bevorzugt werden sowohl am Gerät 1 wie auch am Zweitgerät 16 jeweils Sender 14a und Empfänger 14b angebracht. Das Zweitgerät 16 weist vorzugsweise ebenfalls einen Prozessor 4 auf, um eine Datenreduktion vorzunehmen.

Die Verwendung eines Zweitgerätes 16 trägt zur Verbesserung der aufbereiteten Herzton-Signale 34 hinsichtlich Genauigkeit, Zuverlässigkeit, Empfindlichkeit und/oder Störungsanfälligkeit bei, da mehr Informationen zu den Herztönen zur Verfügung stehen. Zudem können weitere Zusatzgeräte zugeschaltet werden, welche körperrelevante Daten erheben können, wie beispielsweise Activity-Tracker, Smartwatches oder andere sogenannte Wearables, um die Qualität der Daten weiter zu verbessern. Eine andere Möglichkeit zur Verbesserung der Qualität ist die Verwendung eines Kalman Filters zur gewichteten Kombination zweier oder mehrerer aufbereiteter Herzton-Signale 34, und/oder zur Integration der körperrelevanten Daten weiterer Zusatzgeräte.

In der Folge wird das Verfahren zur Erzeugung von aufbereiteten Herzton-Signalen 34 eines Benutzers 2 auf Grund eines akustischen Ausgangssignals 30 beschrieben, mit dem die Herztätigkeit des Benutzers 2 überwacht wird, unter Verwendung eines Gerätes 1. Es umfasst die folgenden Schritte:
a. Positionieren des Gerätes 1 im Bereich eines Ohrs des Benutzers 2;
b. Erzeugung, Verstärkung und Ausgabe eines Referenzsignals 36 als Sinussignal im Ultraschallbereich, in einen Gehörgang des Benutzers 2 mittels eines Tongenerators 18, eines Verstärkers 10 und eines Lautsprechers 11
c. Aufnehmen eines akustischen Ausgangssignals 30 im Gehörgang des Benutzers 2, welches dessen Herztöne 39 und das Referenzsignal umfasst, mit einem Mikrophon 3;
d. Weiterleiten dieses Ausgangssignals 30 an einen Prozessor 4;
e. Erzeugung von aufbereiteten Herzton-Signalen 34 durch Echtzeit Bearbeitung dieses Ausgangssignals 30 im Prozessor 4, indem
   ∘ in einem Vorfilter 6 aus dem Ausgangssignal 30 entstörte Herzton-Signale 32 erzeugt werden,
   ∘ in einem Multiplikator 21 die entstörten Herzton-Signale 32 mit dem Referenzsignal 36 demoduliert werden und so ein Zwischensignal 31 erzeugt wird,
   ∘ in einem Programm zur Bildung von Mittelwerten 7 aus dem Zwischensignal 31 verbesserte Herzton-Signale 35 gebildet werden,
   ∘ und in einem frequenzselektiven Filter 8 aus den verbesserten Herzton-Signalen 35 die aufbereiteten Herzton-Signale 34 erzeugt werden.

Alle diese Schritte werden in Echtzeit ausgeführt. Die aufbereiteten Herzton-Signale 34 werden anschliessend mit mindestens einem Mittel 11, 12 weitergeleitet.

In bevorzugten Verfahren können zudem folgende Schritte, einzeln oder in Kombination miteinander, ausgeführt werden:
Aus den aufbereiteten Herzton-Signalen 34 wird in einem Herzton-Analyseprogramm 9, in welchem Vergleichswerte gespeichert sind, eine Analyse durchgeführt und damit eine individuelle Nachricht erstellt, welche dem Benutzer 2 als zusätzliches Signal 37 über den Verstärker 10 und den Lautsprecher 11 zugespielt wird. Diese Analyse kann insbesondere das Erstellen von Spektren umfassen. Sie kann intern im Gerät oder extern in einer Datenverarbeitungsanlage 13 durchgeführt werden. Dort können sie mit einer App oder mit einem Auswerteprogramm weitere Informationen liefern. Die Nachricht kann beispielsweise Informationen zum Gesundheitszustand und/oder Anweisungen zur sportlichen Betätigung umfassen.

Für die externe Analyse werden die aufbereiteten Herzton-Signale 34 durch eine Ausgabeeinheit 12 auf die externe Datenverarbeitungsanlage 13 übertragen, vorzugsweise kabellos. Dazu können Sender 14a und Empfänger 14b am Gerät verwendet werden.

Zur Verbesserung der Qualität können Daten 38 aus einem Zweitgerät 16 derselben Art und/oder aus einer anderen Datenquelle, beispielsweise von einem Wearable, in den Prozessor 4 und/oder in eine externe Datenverarbeitungsanlage 13 eingelesen und bei einer weiteren Verarbeitung der aufbereiteten Herzton-Signale 34 mitberücksichtigt werden. So werden aus allen eingegangenen Daten 38 durch Vergleiche und weitere Beurteilungen kombinierte aufbereitete Herzton-Signale 34 erzeugt. Auch hier wird unter Qualität Zuverlässigkeit, Empfindlichkeit und/oder Störungsanfälligkeit der Signale verstanden.

Der Datenaustausch 38 mit dem Zweitgerät 16 oder der anderen Datenquelle erfolgt dabei vorzugsweise kabellos mittels Sender und Empfänger 14.

Zudem kann regelmässig überprüft werden, ob Herztöne erkannt werden. Selbst wenn keine Herztöne erkannt werden, kann nach Mustern gesucht werden, um das erfasste akustische Ausgangssignal anderen Quellen zuzuordnen, beispielsweise der Atmung, einem Schnarchen, Kauen oder Erschütterungen, die auf eine sportliche Betätigung hinweisen, oder der Manipulation des Gerätes 1 selbst. Die Erkenntnis über die anderen Quellen der akustischen Ausgangssignale 30 kann im Gerät 1 selbst und/oder in einer externen Datenverarbeitungsanlage 13 ausgewertet und dem Benutzer zur Verfügung gestellt werden. Solche zusätzlichen Informationen können wichtig sein, wenn es das primäre Ziel ist, Herzrhythmusstörungen des Benutzers 2 auszuschliessen oder zu bestätigen.

Die Ermittlung und Analyse solcher anderen Tätigkeiten, die zu den akustischen Ausgangssignalen 30 beitragen, kann im Gerät 1 selbst und/oder in der externen Datenverarbeitungsanlage 13 ausgewertet werden und dem Benutzer in geeigneter Weise zur Verfügung gestellt werden. So kann beispielsweise der Schlaf oder die Bewegungsintensität überwacht werden.

### Bezugszeichenliste

- 1: Gerät
- 2: Benutzer; Ohr des Benutzers, Gehörgang des Ohrs
- 3: Mikrophon
- 4: Prozessor
- 5: Datenspeicher
- 6: Vorfilter
- 7: Programm zur Bildung von Mittelwerten; 7' zweites Programm zur Bildung von Mittelwerten
- 8: Frequenzselektiver Filter
- 9: Herzton-Analyseprogramm
- 10: Verstärker
- 11: Lautsprecher
- 12: Ausgabeeinheit
- 13: Externe Datenverarbeitungsanlage
- 14: Sender und Empfänger; 14a: Sender; 14b: Empfänger
- 15: Energiespeicher
- 16: Zweitgerät
- 17: Zweites Mikrophon
- 18: Tongenerator
- 21: Multiplikator oder Phasenregelschleife
- 22: Erster Signalverstärker
- 23: Zweiter Signalverstärker
- 30: Akustisches Ausgangssignal
- 31: neue Herzton-Signale
- 32: entstörte Herzton-Signale
- 34: Aufbereitete Herzton-Signale
- 35: Verbesserte Herzton-Signale
- 36: Referenzsignal, Tonfolge, Musik, gesprochener Text
- 37: Zusätzliches Signal
- 38: Daten, Datenaustausch zwischen dem Gerät, einem Zweitgerät, und/oder einer externen Datenverarbeitungsanlage
- 39: Modulierte Herztöne

## Patentansprüche

1. Gerät (1) zur Erzeugung von aufbereiteten Herzton-Signalen (34) eines Benutzers (2) auf Grund eines akustischen Ausgangssignals (30), um eine Herztätigkeit des Benutzers (2) analysieren zu können, wobei das Gerät (1) im Bereich eines Ohres des Benutzers (2) tragbar ist, umfassend
- einen Verstärker (10) und einen ausgangsseitig damit verbundenen Lautsprecher (11), zur Ausgabe eines Referenzsignals (36) in einen Gehörgang (2) des Benutzers,
- ein Mikrophon (3) zum Aufnehmen eines Ausgangssignals (30) in diesem Gehörgang des Benutzers (2), welches das Referenzsignal (36) und die damit modulierten Herztöne (39) des Benutzers umfasst, sowie
- einen Prozessor (4) zur Echtzeit-Bearbeitung dieses Ausgangssignals (30), zur Erzeugung von aufbereiteten Herzton-Signalen (34), wobei das Gerät (1) zudem
- einen Tongenerator (18) umfasst zur Erzeugung des Referenzsignals (36), welches ein Sinussignal im Ultraschallbereich ist und sowohl in den Verstärker (10) als auch in den Prozessor (4) geleitet werden kann, wobei im Prozessor (4)
- ein Vorfilter (6) angeordnet ist, welcher aus dem Ausgangssignal (30) entstörte Herzton-Signale (32) erzeugen kann,
- sowie ein Multiplikator (21), der die entstörten Herzton-Signale (32) mit dem Referenzsignal (36) demodulieren und so ein Zwischensignal (31) erzeugen kann,
- sowie ein Programm zur Bildung von Mittelwerten (7), welches aus dem Zwischensignal (31) verbesserte Herzton-Signale (35) bilden kann,
- und einen frequenzselektiven Filter (8), welcher aus den verbesserten Herzton-Signalen (35) die aufbereiteten Herzton-Signale (34) erzeugen kann.

2. Gerät (1) nach Anspruch 1, **dadurch gekennzeichnet, dass** im Prozessor (4) ein erster Signalverstärker (22) zum Verstärken des Referenzsignals (36) sowie ein zweiter Signalverstärker (23) zum Verstärken des entstörtes Herzton-Signals (32) angeordnet sind.

3. Gerät (1) nach Anspruch 2, **dadurch gekennzeichnet, dass** ein zweites Programm (7') zur Bildung von Mittelwerten angeordnet ist, welches Mittelwerte aus den entstörten Herzton-Signalen (32) bilden kann, zur Pegeleinstellung des ersten Signalverstärkers (22).

4. Gerät (1) nach einem der vorhergehenden Ansprüche, **dadurch gekennzeichnet, dass** der Verstärker (10) zudem mit einer Leitung verbunden ist, über die ein zusätzliches Signal (37) eingespeist und über den Lautsprecher (11) abgespielt werden kann.

5. Gerät (1) nach einem der vorhergehenden Ansprüche, **dadurch gekennzeichnet, dass** der Prozessor (4) ausgangsseitig des frequenzselektiven Filters (8) ein Herzton-Analyseprogramm (9) umfasst zur Analyse der aufbereiteten Herzton-Signale (34) auf Grund von gespeicherten Vergleichswerten.

6. Gerät (1) nach Anspruch 5, **dadurch gekennzeichnet, dass** auf Grund dieser Analyse eine individuelle Nachricht erstellt werden kann, welche dem Benutzer (2) über den Verstärker (10) und den Lautsprecher (11) als zusätzliches Signal (37) zugespielt werden kann.

7. Gerät (1) nach einem der vorhergehenden Ansprüche, **gekennzeichnet durch** eine Ausgabeeinheit (12) zur Ausgabe der aufbereiteten Herzton-Signale (34) auf eine externe Datenverarbeitungsanlage (13), welche vorzugsweise einen Sender (14a) umfasst für eine kabellose Übertragung auf die externe Datenverarbeitungsanlage (13).

8. Gerät (1) nach einem der vorhergehenden Ansprüche, **gekennzeichnet durch** einen Datenspeicher (5) und/oder einen Energiespeicher (15), vorzugsweise einen aufladbaren.

9. Gerät (1) nach einem der vorhergehenden Ansprüche, **gekennzeichnet durch** ein Zweitgerät (16) derselben Art, das am zweiten Ohr des Benutzers (2) tragbar ist und ein zweites Mikrophon (17) umfasst, wobei Daten (38) entweder direkt vom Zweitgerät (16) an das Gerät (1) übermittelt oder zwischen ihnen ausgetauscht werden können, oder wobei sowohl das Gerät (1) als auch das Zweitgerät (16) Daten (38) an eine externe Datenverarbeitungsanlage (13) übermitteln können, zur Verbesserung der aufbereiteten Herzton-Signale (34) hinsichtlich Genauigkeit, Zuverlässigkeit, Empfindlichkeit und/oder Störungsanfälligkeit, wobei die Daten (38) zwischen dem Gerät (1), dem Zweitgerät (16) und/oder der externen Datenverarbeitungsanlage (13) vorzugsweise kabellos mittels Sender und Empfänger (14) übermittelt werden können.

10. Gerät (1) nach einem der vorhergehenden Ansprüche, **dadurch gekennzeichnet, dass** es als Hörgerät oder als In-Ear-System ausgestaltet ist.

11. Verfahren zur Erzeugung von aufbereiteten Herzton-Signalen (34) eines Benutzers (2) auf Grund eines akustischen Ausgangssignals (30), um die Herztätigkeit des Benutzers (2) überwachen zu können, unter Verwendung eines Gerätes (1) gemäss einem der vorhergehenden Ansprüche, mit den folgenden Schritten:
a. Positionieren des Gerätes (1) im Bereich eines Ohrs des Benutzers (2);
b. Erzeugung, Verstärkung und Ausgabe eines Referenzsignals (36) als Sinussignal im Ultraschallbereich, in einen Gehörgang des Benutzers (2) mittels eines Tongenerators (18), eines Verstärkers (10) und eines Lautsprechers (11)
c. Aufnehmen eines akustischen Ausgangssignals (30) im Gehörgang des Benutzers (2), welches dessen Herztöne (39) und das Referenzsignal umfasst, mit einem Mikrophon (3);
d. Weiterleiten dieses Ausgangssignals (30) an einen Prozessor (4);
e. Erzeugung von aufbereiteten Herzton-Signalen (34) durch Echtzeit Bearbeitung dieses Ausgangssignals (30) im Prozessor (4), indem
∘ in einem Vorfilter (6) aus dem Ausgangssignal (30) entstörte Herzton-Signale (32) erzeugt werden,
∘ in einem Multiplikator (21) die entstörten Herzton-Signale (32) mit dem Referenzsignal (36) demoduliert werden und so ein Zwischensignal (31) erzeugt wird,
∘ in einem Programm zur Bildung von Mittelwerten (7) aus dem Zwischensignal (31) verbesserte Herzton-Signale (35) gebildet werden,
∘ und in einem frequenzselektiven Filter (8) aus den verbesserten Herzton-Signalen (35) die aufbereiteten Herzton-Signale (34) erzeugt werden.

12. Verfahren nach Anspruch 11, **dadurch gekennzeichnet, dass** aus den aufbereiteten Herzton-Signalen (34) in einem Herzton-Analyseprogramm (9), in dem Vergleichswerte gespeichert sind, eine Analyse durchgeführt und damit eine individuelle Nachricht erstellt wird, welche dem Benutzer (2) als zusätzliches Signal (37) über den Verstärker (10) und den Lautsprecher (11) zugespielt wird.

13. Verfahren nach Anspruch 11 oder 12, **dadurch gekennzeichnet, dass** die aufbereiteten Herzton-Signale (34) durch eine Ausgabeeinheit (12) auf eine externe Datenverarbeitungsanlage (13) übertragen werden, vorzugsweise kabellos.

14. Verfahren nach einem der Ansprüche 11 bis 13, **dadurch gekennzeichnet, dass** zur Verbesserung der Qualität Daten (38) aus einem Zweitgerät (16) derselben Art und/oder aus einer anderen Datenquelle in den Prozessor (4) und/oder in eine externe Datenverarbeitungsanlage (13) eingelesen und bei einer weiteren Verarbeitung der aufbereiteten Herzton-Signale (34) mitberücksichtigt werden, wobei der Datenaustausch (38) mit dem Zweitgerät (16) oder der anderen Datenquelle vorzugsweise kabellos mittels Sender und Empfänger (14) durchgeführt wird.

15. Verfahren nach einem der Ansprüche 11 bis 14, **dadurch gekennzeichnet, dass** regelmässig überprüft wird, ob Herztöne erkannt werden, und dass in dem Fall, selbst wenn keine Herztöne erkannt werden, nach Mustern gesucht wird, um das erfasste akustische Ausgangssignal anderen Quellen zuzuordnen, beispielsweise der Atmung, einem Schnarchen, Kauen oder Erschütterungen, die auf eine sportliche Betätigung hinweisen, oder der Manipulation des Gerätes 1 selbst, wobei vorzugsweise die Erkenntnis über die anderen Quellen der akustischen Ausgangssignale (30) im Gerät (1) selbst und/oder in einer externen Datenverarbeitungsanlage (13) ausgewertet und dem Benutzer zur Verfügung gestellt wird.

## Claims

1. A device (1) for generating prepared heart sound signals (34) of a user (2) on the basis of an acoustic output signal (30) in order to be able to analyse the cardiac activity of the user (2), wherein the device (1) is wearable in the region of an ear of the user (2), said device comprising
- an amplifier (10) and a loudspeaker (11) connected thereto on the output side thereof, to output a reference signal (36) into the auditory canal (2) of the user;
- a microphone (3) for receiving an output signal (30) in said auditory canal of the user (2), said output signal comprising the reference signal (36) and the heart sounds (39) of the user modulated therewith; and
- a processor (4) for real-time processing of this output signal (30), to generate prepared heart sound signals (34),
wherein the device (1) also comprises
- a sound generator (18) for generating the reference signal (36), which is a sinusoidal signal in the ultrasound range and can be conducted both into the amplifier (10) and into the processor (4),
wherein in the processor (4)
- there is arranged a prefilter (6), which can generate interference-suppressed heart sound signals (32) from the output signal (30),
- and a multiplier (21), which can demodulate the interference-suppressed heart sound signals (32) with the reference signal (36) and can thus generate an intermediate signal (31),
- and a program for forming mean values (7), which can form improved heart sound signals (35) from the intermediate signal (31),
- and a frequency-selective filter (8), which can generate the prepared heart sound signals (34) from the improved heart sound signals (35).

2. The device (1) according to claim 1, **characterised in that** a first signal amplifier (22) for amplifying the reference signal (36) and a second signal amplifier (23) for amplifying the interference-suppressed heart sound signal (32) are disposed in the processor (4).

3. The device (1) according to claim 2, **characterised in that** a second program (7') for forming mean values is provided, which can form mean values from the interference-suppressed heart sound signals (32) to set the level of the first signal amplifier (22).

4. The device (1) according to any one of the preceding claims, **characterised in that** the amplifier (10) is additionally connected to a line, by means of which an additional signal (37) is fed in and can be played back by means of the loudspeaker (11).

5. The device (1) according to any one of the preceding claims, **characterised in that** the processor (4) comprises a heart sound analysis program (9) on the output side of the frequency-selective filter (8) for analysis of the prepared heart sound signals (34) on the basis of stored comparison values.

6. The device (1) according to claim 5, **characterised in that** an individual message can be created on the basis of this analysis and can be passed to the user (2) by means of the amplifier (10) and the loudspeaker (11) as an additional signal (37).

7. The device (1) according to any one of the preceding claims, **characterised by** an output unit (12) for outputting the prepared heart sound signals (34) at an external data processing system (13), which preferably comprises a transmitter (14a) for wireless transfer to the external data processing system (13).

8. The device (1) according to any one of the preceding claims, **characterised by** a data store (5) and/or an energy store (15), preferably a rechargeable one.

9. The device (1) according to any one of the preceding claims, **characterised by** a second device (16) of the same kind, which is wearable on the second ear of the user (2) and comprises a second microphone (17), wherein data (38) can be transmitted either directly from the second device (16) to the device (1) or can be exchanged therebetween, or wherein both the device (1) and the second device (16) can transmit data (38) to an external data processing system (13) to improve the prepared heart sound signals (34) in respect of accuracy, reliability, sensitivity and/or susceptibility to interference, wherein the data (38) can be transmitted between the device (1), the second device (16) and/or the external data processing system (13) preferably wirelessly by means of transmitters and receivers (14).

10. The device (1) according to any one of the preceding claims, **characterised in that** it is formed as a hearing aid or as an in-ear system.

11. A method for generating prepared heart sound signals (34) of a user (2) on the basis of an acoustic output signal (30) in order to be able to analyse the cardiac activity of the user (2), using a device (1) according to any one of the preceding claims, said method comprising the following steps:
a. positioning the device (1) in the region of an ear of the user (2);
b. generating, amplifying and outputting a reference signal (36) as a sinusoidal signal in the ultrasound range in an auditory canal of the user (2) by means of a sound generator (18), an amplifier (10) and a loudspeaker (11),
c. recording, by means of a microphone (3), an acoustic output signal (30) in the auditory canal of the user (2), said output signal comprising heart sounds of said user (39) and the reference signal;
d. forwarding this output signal (30) to a processor (4) ;
e. generating prepared heart sound signals (34) by real-time processing of said output signal (30) in the processor (4), by
∘ generating, in a pre-filter (6), interference-suppressed heart sound signals (32) from the output signal (30),
∘ demodulating, in a multiplier (21), the interference-suppressed heart sound signals (32) with the reference signal (36) and thus generating an intermediate signal (31),
∘ forming improved heart sound signals (35) in a program for forming mean values (7) from the intermediate signal (31),
∘ and generating the prepared heart sound signals (34) in a frequency-selective filter (8) from the improved heart sound signals (35) .

12. The method according to claim 11, **characterised in that** an analysis is performed on the basis of the prepared heart sound signals (34) in a heart sound analysis program (9), in which comparison values are stored, and an individual message is thus created, which is played to the user (2) as an additional signal (37) by means of the amplifier (10) and the loudspeaker (11).

13. The method according to claim 11 or 12, **characterised in that** the prepared heart sound signals (34) are transferred by an output unit (12) to an external data processing system (13), preferably wirelessly.

14. The method according to any one of claims 11 to 13, **characterised in that**, to improve the quality, data (38) from a second device (16) of the same kind and/or from another data source are fed into the processor (4) and/or into an external data processing system (13) and are taken into consideration in a further processing of the prepared heart sound signals (34), wherein the data exchange (38) with the second device (16) or the other data source is performed preferably wirelessly by means of transmitters and receivers (14).

15. The method according to any one of claims 11 to 14, **characterised in that** it is regularly checked whether heart sounds have been identified, and **in that**, in this case, even if no heart sounds have been identified, patterns are looked for in order to assign the detected acoustic output signal to other sources, for example breathing, snoring, chewing or vibrations indicating a sporting activity, or manipulation of the device (1) itself, wherein the recognition of the other sources of the acoustic output signals (30) is preferably evaluated in the device (1) itself and/or in an external data processing system (13) and is made available to the user.

## Revendications

1. Appareil (1) de génération de signaux de battements cardiaques traités (34) d'un utilisateur (2) sur la base d'un signal acoustique de sortie (30) afin de pouvoir analyser une activité cardiaque de l'utilisateur (2), l'appareil (1) étant portable au niveau d'une oreille de l'utilisateur (2), comprenant :
- un amplificateur (10) et un haut-parleur qui lui est connecté au niveau de la sortie (11) pour délivrer un signal de référence (36) dans un conduit auditif (2) de l'utilisateur,
- un micro (3) pour recevoir dans ce conduit auditif de l'utilisateur (2) un signal de sortie (30) qui comprend le signal de référence (36) et les battements cardiaques ainsi modulés (39) de l'utilisateur, et
- un processeur (4) pour le traitement en temps réel de ce signal de sortie (30) afin de générer des signaux de battements cardiaques traités (34),
l'appareil (1) comprenant en outre
- un générateur de son (18) pour générer le signal de référence (36) qui est un signal sinusoïdal dans la plage ultrasonique et qui peut être conduit aussi bien dans l'amplificateur (10) que dans le processeur (4),
dans lequel, dans le processeur (4),
- est disposé un préfiltre (6) qui peut générer des signaux de battements cardiaques antiparasités (32) à partir du signal de sortie (30),
- ainsi qu'un multiplicateur (21) qui peut démoduler les signaux de battements cardiaques antiparasités (32) avec le signal de référence (36) et ainsi générer un signal intermédiaire (31),
- ainsi qu'un programme de calcul de valeurs moyennes (7) qui peut former des signaux de battements cardiaques améliorer (35) à partir du signal intermédiaire (31), et
- un filtre sélectif de fréquence (8) qui peut générer les signaux de battements cardiaques traités (34) à partir des signaux de traitement cardiaque améliores (35).

2. Appareil (1) selon la revendication 1, **caractérisé en ce que**, dans le processeur (4) sont disposés un premier amplificateur de signaux (22) pour l'amplification du signal de référence (36) et un second amplificateur de signaux (23) pour amplifier le signal de battements cardiaques antiparasité (32).

3. Appareil (1) selon la revendication 2, **caractérisé en ce qu'**est disposé un second programme (7') pour le calcul de valeurs moyennes, qui peut calculer des valeurs moyennes à partir des signaux de battements cardiaques antiparasités (32) pour le réglage du niveau du premier amplificateur de signaux (22).

4. Appareil (1) selon une des revendications précédentes, **caractérisé en ce que** l'amplificateur (10) est en outre connecté à une ligne grâce à laquelle un signal supplémentaire (37) peut être engrangé et diffusé par le haut-parleur (11).

5. Appareil (1) selon une des revendications précédentes, **caractérisé en ce que** le processeur (4) comprend, du côté sortie du filtre sélectif de fréquence (8), un programme d'analyse de battements cardiaques (9) pour l'analyse des signaux de battements cardiaques traités (34) sur la base de valeurs comparatives enregistrées.

6. Appareil (1) selon la revendication 5, **caractérisé en ce que**, sur la base de cette analyse, il peut être établi un message individuel qui peut être diffusé à l'utilisateur (2) par l'intermédiaire de l'amplificateur (10) et du haut-parleur (11) sous forme d'un signal supplémentaire (37).

7. Appareil (1) selon une des revendications précédentes, **caractérisé par** une unité de diffusion (12) pour la diffusion des signaux de battements cardiaques traités (34) vers une installation externe de traitement de données (13) qui comprend de préférence un émetteur (14a) pour un transfert sans câble à l'unité externe de traitement de données (13).

8. Appareil (1) selon une des revendications précédentes, **caractérisé par** une mémoire de données (5) et/ou un accumulateur d'énergie (15), de préférence chargeable.

9. Appareil (1) selon une des revendications précédentes, **caractérisé par** un appareil secondaire (16) du même type qui est portable à la seconde oreille de l'utilisateur (2) et comprend un second micro (17), les données (38) pouvant être soit transmises directement par l'appareil secondaire (16) à l'appareil (1), soit échangées entre eux, et aussi bien l'appareil (1) que l'appareil secondaire (16) pouvant transmettre des données (38) à une installation externe de traitement de données (13) pour améliorer les signaux de battements cardiaques traités (34) au niveau de la précision, de la fiabilité, de la sensibilité et/ou de la tendance au parasitage, les données (38) pouvant de préférence être transférées entre l'appareil (1), l'appareil secondaire (16) et/ou l'installation externe de traitement de données (13) sans câble au moyen de l'émetteur et du récepteur (14).

10. Appareil (1) selon une des revendications précédentes, **caractérisé en ce qu'**il est conçu comme un appareil auditif ou un système intégré dans l'oreille.

11. Procédé de génération de signaux de battements cardiaques traités (34) d'un utilisateur (2) sur la base d'un signal acoustique de sortie (30) afin de pouvoir surveiller l'activité cardiaque de l'utilisateur (2), en utilisant un appareil (1) selon une des revendications précédentes, comportant les étapes suivantes :
a. positionnement de l'appareil (1) au niveau de l'oreille de l'utilisateur (2) ;
b. génération, amplification et émission d'un signal de référence (36) sous forme d'un signal sinusoïdal dans la plage ultrasonore, dans un conduit auditif de l'utilisateur (2) au moyen d'un générateur de son (18), d'un amplificateur (10) et d'un haut-parleur (11) ;
c. réception dans le conduit auditif de l'utilisateur (2) d'un signal de sortie acoustique (30) qui comprend ses battements cardiaques (39) et le signal de référence au moyen d'un micro (3) ;
d. retransmission du signal de sortie (30) à un processeur (4) ;
e. génération de signaux de battements cardiaques traités (34) par traitement en temps réel de ce signal de sortie (30) dans le processeur (4) par le fait que,
∘ dans un préfiltre (6), des signaux de battements cardiaques antiparasités (32) sont générés à partir du signal de sortie (30),
∘ dans un multiplicateur (21), les signaux de battements cardiaques antiparasités (32) sont démodulés avec le signal de référence (36) et qu'un signal intermédiaire (31) est ainsi généré,
∘ dans un programme de calcul de valeurs moyennes (7), des signaux de battements cardiaques améliorés (35) sont créés à partir du signal intermédiaire (31),
∘ et que, dans un filtre sélectif de fréquence (8), les signaux de battements cardiaques traités (34) sont générés à partir des signaux de battements cardiaques améliorés (35).

12. Procédé selon la revendication 11, **caractérisé en ce que**, à partir des signaux de battements cardiaques traités (34), dans un programme d'analyse de battements cardiaques (9) dans lequel des valeurs correctives sont enregistrées, une analyse est réalisée et il est ainsi établi un message individuel qui est diffusé à l'utilisateur (2) sous forme d'un signal supplémentaire (37) par l'amplificateur (10) et le haut-parleur (11).

13. Procédé selon la revendication 11 ou 12, **caractérisé en ce que** les signaux de battements cardiaques traités (34) sont transmis par une unité d'émission (12) à une installation externe de traitement de données (13), de préférence sans câble.

14. Procédé selon une des revendications 11 à 13, **caractérisé en ce que**, pour améliorer la qualité, des données (38) sont lues dans un appareil secondaire (16) de même type et/ou une autre source de données dans le processeur (4) et/ou dans une installation externe de traitement de données (13) et prises en compte lors d'un traitement ultérieur des signaux de battements cardiaques traités (34), l'échange de données (38) avec l'appareil secondaire (16) ou l'autre source de données étant de préférence réalisé sans câble au moyen d'un émetteur et d'un récepteur (14).

15. Procédé selon une des revendications 11 à 14, **caractérisé en ce qu'**il est vérifié régulièrement si on détecte des battements cardiaques et que, dans le cas, où on ne détecte même pas de battements cardiaques, on cherche des échantillons pour affecter le signal de sortie acoustique détecté à d'autres sources, par exemple la respiration, un ronflement, une mastication ou des secousses qui sont l'indice d'une activité sportive ou la manipulation de l'appareil 1 lui-même, la détection par d'autres sources des signaux de sortie acoustique (30) étant de préférence évaluée et mise à disposition de l'utilisateur dans l'appareil (1) lui-même et/ou dans une installation externe de traitement de données (13).
